# FASCICULE DE BREVET EUROPEEN

(11) **EP 0 850 650 B1**
(45) Date de publication et mention de la délivrance du brevet: **23.04.2003**
(21) Numéro de dépôt: 97403191.6
(22) Date de dépôt: 30.12.1997
(51) Int. Cl.: A61L 24/10

(54) **Procédé de préparation d'une colle biologique capable de coaguler par simple addition d'ions calcium**
Verfahren zur Herstellung eines Gewebeklebers, dessen Koagulation durch Zugabe von Calciumionen erfolgt
Process for preparing a fibrin glue capable of coagulating by addition of calcium ions

(30) Priorité: 30.12.1996 FR 9616214
(43) Date de publication de la demande: 01.07.1998
(73) Titulaire: BIO HOLDINGS INTERNATIONAL LIMITED, Tortola (VG)
(72) Inventeur: Patat, Jean-Louis, 75017 Paris (FR); Delmas, Olivier, 37250 Montbazon (FR); Schmitthaeusler, Roland, 78180 Montigny Le Bretonneux (FR)
(74) Mandataire: Hubert, Philippe

(56) Documents cités:
- EP-A- 0 592 242
- WO-A-93/19805
- PATENT ABSTRACTS OF JAPAN vol. 006, no. 122 (C-112), 7 juillet 1982 & JP 57 050923 A (ONO PHARMACEUT CO LTD), 25 mars 1982,

## Description

L'invention a pour objet un procédé de préparation d'une colle biologique capable de coaguler par simple addition d'ions calcium.

On sait que des concentrés de protéines contenant notamment du fibrinogène et du facteur XIII peuvent coaguler par addition de thrombine en donnant un réseau de fibrine, et sont utilisés comme matériaux collants. Plus précisément, sous l'effet de la thrombine, le fibrinogène est transformé en monomère de fibrine. Les monomères de fibrine s'assemblent pour former des polymères de fibrine. Sous l'action du facteur XIII activé par la thrombine, en présence d'ions calcium, les chaînes de fibrine sont réticulées. Ainsi, l'addition de thrombine, suivie de l'application du mélange résultant, provoque une coagulation par formation de fibrine sur les parties à coller, selon un mécanisme reproduisant la phase finale de la coagulation sanguine. Les concentrés de fibrinogène constituent donc, en coagulant, un matériau collant permettant notamment de réunir et de maintenir accolés des tissus vivants tout en exerçant une action hémostatique ; voir par exemple le brevet FR-2448900. De tels matériaux collants sont couramment appelés "colles biologiques" ou "colles de fibrine" et sont utilisés en chirurgie, notamment pour prévenir ou arrêter les saignements, remplacer les fils de sutures ou renforcer celles-ci, maintenir en place des greffes, par exemple des greffes de derme, rapprocher les tissus ayant subi une résection, par exemple en chirurgie du poumon ou du tube digestif, ou encore pour coller des pièces de prothèses, etc.

Pour utiliser les colles biologiques, il convient de disposer de thrombine qui est préparée à partir de mélanges de plasmas sanguins, humains ou animaux. Les produits homologues, c'est-à-dire les produits d'origine humaine pour lesquels le donneur et le receveur sont différents, présentent un risque de contamination du receveur par des agents pathogènes du donneur. Ce risque peut être réduit par la sélection des donneurs et la recherche de la présence chez le donneur de marqueurs d'agents pathogènes, ou encore par des traitements physico-chimiques visant à détruire ou à diminuer la virulence des produits préparés. Cependant, il ne peut y avoir aucune certitude quant à l'innocuité des produits homologues.

Les produits préparés à partir de tissus animaux présentent également des risques liés à l'existence d'agents pathogènes transmissibles à l'homme, et aussi des risques d'immunisation et, par suite, de réactions anaphylactiques.

Les risques mentionnés plus haut seraient évités si l'on utilisait uniquement des produits autologues, c'est-à-dire des produits préparés uniquement à partir d'un prélèvement effectué sur le futur receveur.

Le principe de la transfusion autologue est connu et largement répandu, y compris en ce qui concerne la préparation de colles à base de fibrinogène. Toutefois, les colles dites "autologues" ne le sont, en réalité, qu'en ce qui concerne la solution de fibrinogène. En effet, la solution de thrombine est obtenue à partir de plasmas homologues ou hétérologues, mais non de façon autologue ; voir notamment CEDERHOLM-WILLIAMS, Lancet 344, 336-337 (1994).

Dans la demande de brevet PCT WO94/07548, on a décrit une colle biologique enrichie en facteurs plaquettaires, qui est capable de coaguler sans addition de thrombine, par addition à la colle recalcifiée d'un activateur de la phase contact de la coagulation sanguine, tel que du kaolin. Toutefois, dans cette demande de brevet, l'activateur de la phase contact est incorporé au moment de l'emploi de la colle, ce qui rend la mise en oeuvre de l'activation aléatoire et malaisée car le concentré de fibrinogène constitue un produit très visqueux, de manipulation difficile. Il en résulte que le temps de coagulation n'est pas facile à maîtriser. En outre, la coagulation progressant en même temps que l'activation, il est difficile de séparer l'activateur de la colle activée.

On a maintenant découvert qu'il est possible d'obtenir une colle biologique coagulable par simple addition d'ions calcium, sans addition d'un activateur de la phase contact au moment de l'emploi de la colle, et sans addition de thrombine ou de prothrombine avant l'emploi ou au moment de l'emploi. On a découvert en effet qu'il est possible de préactiver le plasma servant à la préparation de la colle biologique, avant d'effectuer les opérations d'obtention du concentré de fibrinogène contenant des facteurs de coagulation, et cela sans qu'on observe une coagulation au cours de la préparation de la colle.

On a découvert en particulier qu'il suffit de préactiver le plasma avec un activateur de la phase contact de la coagulation, pendant un temps suffisant pour activer partiellement la prékallicréine en kallicréine, et le plasma ainsi traité permet d'obtenir ensuite, sans coagulation spontanée, une colle biologique capable de coaguler de façon relativement rapide, par simple recalcification, sans addition de thrombine.

Le brevet US-4.427.650 décrit une colle de fibrine sous forme de poudre contenant un mélange de fibrinogène et de thrombine et/ou de prothrombine. Une telle colle n'est soumise à aucune mesure d'activation et ne contient donc pas de facteurs de coagulation activés non dépendants des ions calcium.

Le document WO91/09641 décrit une colle de fibrine contenant du fibrinogène et de la thrombine ajoutée. Cette colle est préparée de façon que l'activité de la thrombine soit inhibée. Selon un mode de réalisation particulier, cette colle est exempte d'ions calcium, ceux-ci n'étant ajoutés qu'au moment de l'emploi. Cependant, une telle colle, qui contient de la thrombine ajoutée, coagule spontanément, même sans addition d'ions calcium, au bout de 90 secondes environ. Par addition d'ions calcium, elle coagule en moins de 2 secondes. Dans d'autres modes de réalisation, on retarde la coagulation de la colle en l'acidifiant à pH inférieur à 5,5, ce qui inhibe l'activité de la thrombine, et on utilise au moment de l'emploi des moyens permettant d'augmenter le pH, pour annuler l'effet d'inhibition. Selon un autre mode de réalisation, on ajoute à la colle, à l'abri de la lumière, un inhibiteur de thrombine photosensible qui est désactivé à la lumière. Une telle colle, quels que soient ses modes de réalisation, ne contient pas de facteurs de coagulation activés non dépendants des ions calcium.

La colle obtenue selon l'invention est au contraire une colle stable qui, sous forme de solution aqueuse, ne coagule pas spontanément à température ambiante, même à la lumière, et qui est capable de coaguler par simple addition d'ions calcium sans modification de pH. Elle contient des facteurs de coagulation activés non dépendants des ions calcium.

La colle biologique obtenue selon l'invention est une colle préactivée contenant du fibrinogène et au moins un facteur de coagulation activé dont l'activation ne dépend pas des ions calcium. Elle est stable en solution aqueuse, c'est-à-dire que la solution ne coagule pas de façon spontanée, pendant au moins une heure à une température de 20°C, et elle est capable de coaguler en moins de 5 minutes par simple addition d'ions calcium, sans mise en contact avec un activateur.

Ainsi, la colle biologique obtenue selon l'invention est capable de coaguler sans addition de thrombine ni de prothrombine.

Cette colle peut se présenter sous la forme d'une solution aqueuse. Elle peut également se présenter sous la forme d'un lyophilisat à partir duquel on peut reconstituer une solution aqueuse au moment de l'emploi.

Les mécanismes de la coagulation sanguine sont pour l'essentiel connus. On sait notamment que l'un des mécanismes d'activation initiale de la coagulation peut être facilement reproduit in vitro en mettant en contact le plasma avec un activateur, qui peut être un solide insoluble ayant une surface chargée négativement, comme par exemple le verre ou le kaolin, ou un activateur soluble, à l'état dissous. Cette activation initiale résulte de l'interaction entre plusieurs protéines plasmatiques : le facteur XII, le facteur XI, la prékallicréine et le kininogène de haut poids moléculaire (KHPM) ; voir par exemple WACHTFOGEL et al., Thrombosis Research, 72, 1-21 (1993). Au contact de la surface activatrice, la conformation spatiale du facteur XII subit une modification. Le facteur XII devient alors capable d'activer la prékallicréine en kallicréine, cette réaction étant amplifiée par le KHPM. La kallicréine ainsi formée agit sur le facteur XII et le transforme en facteur XII activé (ou facteur XIIa). Le facteur XIIa possède la propriété d'activer le facteur XI.

Ces réactions impliquant le facteur XII, la prékallicréine, le KHPM et le facteur XI sont appelées "réactions du système contact", et les facteurs de coagulation impliqués sont appelés "facteurs du système contact" ou "facteurs contact".

La phase de la coagulation pendant laquelle se produisent les réactions du système contact est appelée "phase contact".

Les mécanismes aboutissant à la coagulation impliquent encore : l'activation du facteur IX par le facteur XIa, cette activation nécessitant la présence d'ions calcium ; l'activation du facteur X sous l'action d'un complexe formé par les facteurs IXa, VIIIa et F3P (facteur 3 plaquettaire), en présence d'ions calcium ; l'activation de la prothrombine en thrombine sous l'influence d'un complexe des facteurs Xa, Va et F3P, en présence d'ions calcium. La thrombine permet la transformation du fibrinogène en fibrine dite "soluble", et permet également, en présence d'ions calcium, l'activation du facteur XIII. Le facteur XIII activé permet la transformation de la fibrine soluble en fibrine insoluble qui forme le caillot sanguin.

On sait qu'une autre voie de la coagulation, appelée voie extrinsèque, comporte l'activation du facteur VII sous l'action de la thromboplastine tissulaire. Le facteur VII activé est capable notamment d'activer le facteur X, et aussi le facteur IX. En outre, il est connu que le facteur XII activé est capable d'activer le facteur VII. Ainsi, l'activation du facteur VII peut résulter de l'activation de la phase contact.

L'invention repose notamment sur la découverte du fait qu'en activant partiellement les facteurs du système contact, par exemple sur le plasma de départ, et en tout cas avant l'obtention du concentré de fibrinogène constituant la colle, on peut obtenir finalement une colle biologique capable de coaguler rapidement, par simple addition d'ions calcium, sans ajout de thrombine, ni de prothrombine. On a constaté qu'une telle activation partielle faite à un stade précoce n'entraîne pas de coagulation prématurée, spontanée, lors des opérations consécutives d'obtention du concentré de fibrinogène constituant la colle biologique. Plus précisément, on a découvert qu'il suffit de mettre le plasma en contact avec l'activateur en quantité et pendant un temps suffisants pour que le plasma contienne au moins 15, et en particulier au moins 20, unités de kallicréine par litre. La colle biologique obtenue avec un tel plasma préactivé est alors capable de coaguler en moins de 5 minutes, sans addition d'un activateur et sans addition de thrombine ni de prothrombine, par simple recalcification.

L'unité de kallicréine utilisée ici est définie de la façon suivante : c'est la quantité capable d'hydrolyser une solution 0,52 mM de D-Prolyl-L-Phénylalanyl-L-Arginine-p-nitro-anilide en solution tampon véronal-acétate de sodium de pH 7,35, à 37°C, à la vitesse initiale de 1 µmol/min.

La colle biologique obtenue selon l'invention est caractérisée notamment par le fait qu'elle contient au moins un facteur de coagulation activé, dont l'activation ne dépend pas des ions calcium, choisi parmi le facteur XIIa, le facteur XIa, le facteur VIIa et la kallicréine. Ces facteurs activés peuvent être détectés et/ou dosés par exemple selon les méthodes suivantes :
Facteur XIIa (également appelé "activateur de prékallicréine") : Pharmacopée européenne, 1994, chapitre V.2.1.11 ;
Facteur VIIa : méthode de MORRISSEY et al., Blood, Vol.81, pp.734-744 (1993) on peut utiliser notamment la trousse de dosage commerciale Staclot VIIa-rTF, Diagnostica Stago Réf.00281 ;
Facteur XIa : méthode chronométrique de GYÖNGYÖSSI-ISSA MIC et al., Vox Sang., Vol.70, pp.76-85 (1996) ; ou méthode chromogénique de SCOTT C.F. et COLMAN R.W., Proc. Natl. Acad. Sci. USA, Vol.89, pp.11189-11193 (1992) ;
Kallicréine : voir la partie expérimentale ci-après.

La stabilité de la colle biologique obtenue selon l'invention, attestée par l'absence de coagulation spontanée à température ambiante, montre qu'elle est pratiquement exempte de thrombine. La colle biologique obtenue selon l'invention est en outre pratiquement exempte de fibrine, comme l'atteste sa faible teneur en fibrinopeptide A (en général, à l'issue du procédé de l'invention, moins de 0,5% en poids du fibrinogène présent dans le plasma de départ a été clivé avec formation de fibrinopeptide A ; voir la partie expérimentale ci-après). En revanche, cette colle biologique contient, sous forme non activée, les facteurs de coagulation dont l'activation dépend des ions calcium. Elle contient donc en particulier, outre la prothrombine, les facteurs XIII, X, V et FP3. Généralement, elle contient en outre les facteurs VIII et IX, qui sont nécessaires à la coagulation en l'absence de facteur VII activé.

L'invention a donc pour objet un procédé de préparation d'une colle biologique telle que définie précédemment. C'est un procédé comprenant les étapes consistant à : a) obtenir un plasma sanguin contenant un agent capable de complexer les ions calcium, b) obtenir à partir dudit plasma, selon les méthodes connues, une colle biologique à base de fibrinogène et de facteurs de coagulation, et c) si désiré, soumettre la colle biologique obtenue à une lyophilisation, et dans lequel avant ou pendant l'étape b), on met en contact ledit plasma avec un activateur de la phase contact.

Bien entendu, on peut déterminer une fois pour toutes les conditions de quantité et de durée d'application de l'activateur qui permettent d'obtenir une concentration d'au moins 15 unités de kallicréine, ou, plus simplement, qui permettent d'obtenir une colle biologique conforme à l'invention, c'est-à-dire capable de coaguler en moins de 5 minutes par simple addition d'ions calcium. Il n'y a donc pas de nécessité de doser la kallicréine à chaque fois que l'on met en oeuvre le procédé.

L'étape de mise en contact du plasma avec l'activateur est effectuée de préférence à une température inférieure à 10°C. On opère par exemple à une température de 1 à 8°C et en particulier à 4°C environ.

Lorsque l'activateur est insoluble, on peut si désiré séparer le plasma préactivé de l'activateur. Pour cela, après un temps de contact suffisant, on sépare et élimine l'activateur, par exemple par filtration. Le temps de contact suffisant pour obtenir une transformation au moins partielle de prékallicréine en kallicréine dépend évidemment de la nature de l'activateur, et de la quantité d'activateur. Ce temps peut être déterminé dans chaque cas par des expériences de simple routine ; voir par exemple la partie expérimentale ci-après.

Les agents capables de complexer les ions calcium sont connus. Il s'agit par exemple des sels d'acide citrique comme le citrate de sodium, ou d'un agent chélatant tel qu'un sel d'EDTA (par exemple un sel de sodium de l'EDTA)

Le plasma de départ peut contenir aussi un sel soluble de zinc, tel que le sulfate ou l'acétate, de façon à obtenir par exemple une concentration en ions zinc pouvant aller de 10⁻⁴ M à 10⁻² M environ. On sait que les ions zinc agissent comme accélérateurs des réactions du système contact.

Le contact entre le plasma et l'activateur peut être effectué, dans le cas d'un activateur solide insoluble, dans un conteneur approprié. L'activateur est introduit dans le conteneur, avant ou après l'introduction du plasma. L'activateur peut être fixé à la paroi interne du conteneur. Par exemple, la paroi interne peut être revêtue d'activateur. Lorsque l'activateur n'est pas fixé à la paroi interne du conteneur, il convient de prévoir une étape dans laquelle on sépare, après un temps de contact suffisant, l'activateur et le plasma activé. Pour cela, par exemple, le conteneur peut être muni, intérieurement ou extérieurement, d'un filtre permettant de laisser passer le plasma tout en retenant l'activateur, de façon à séparer le plasma activé de l'activateur après l'étape de mise en contact.

Selon un autre mode de réalisation, on effectue ladite mise en contact en faisant circuler ledit plasma dans une colonne contenant l'activateur. On peut par exemple faire circuler ledit plasma dans la colonne jusqu'à un orifice de sortie, ledit orifice de sortie étant muni d'un filtre permettant de retenir l'activateur. On recueille alors, après un temps de contact suffisant, le plasma activé, tandis que le filtre retient l'activateur.

Lorsque l'obtention de la colle biologique comprend une étape consistant à concentrer le plasma, par exemple à l'aide d'une membrane laissant passer l'eau et les constituants de faible masse moléculaire, mais retenant les protéines, on peut mettre en contact le plasma avec l'activateur pendant cette étape de concentration. Si l'activateur est un solide insoluble, il faudra ensuite le séparer, par exemple par filtration, du plasma concentré ou partiellement concentré.

Les activateurs de la phase contact de la coagulation sont bien connus. Il s'agit notamment de produits solides dont la surface est chargée négativement, tels que le verre, le kaolin, la célite, l'oxyde de zinc, le carbonate de zinc, etc.

On peut également utiliser des activateurs solubles tels que par exemple le sulfate de dextran et l'acide ellagique.

On a également découvert que d'autres produits, tels que le carbonate de calcium (aragonite ou calcite) sont capables d'activer le système contact. On peut donc utiliser un activateur constitué essentiellement d'aragonite, par exemple sous la forme de particules de corail. On peut utiliser par exemple des particules ayant des dimensions de 0,3-2 mm.

On a constaté que l'activation par le carbonate de calcium augmente légèrement la concentration en calcium du plasma, mais de façon suffisamment faible pour ne pas provoquer de coagulation prématurée lors de la préparation de la colle biologique.

On peut encore utiliser des activateurs solubles immobilisés sur des supports solides par des techniques usuelles, par exemple par covalence ou par affinité. De tels supports sur lesquels sont immobilisés des activateurs solubles sont assimilables à des activateurs insolubles et sont utilisés comme ceux-ci.

Les activateurs utilisés dans le procédé de l'invention ne doivent ni retenir en proportion notable (dans le cas d'un activateur solide), ni inhiber, les facteurs de coagulation dont l'activation dépend des ions calcium et qui sont nécessaires à la suite de réactions conduisant à la formation de fibrine et à la coagulation de celle-ci. Les facteurs qui sont nécessaires à cette suite de réactions sont essentiellement, outre la prothrombine, les facteurs V, VIII, IX, X, FP3 et XIII.

En fait, la sélection des activateurs qui conviennent peut être facilement effectuée par des expériences de routine : un activateur convient s'il permet d'obtenir une colle biologique conforme à la définition donnée précédemment, c'est-à-dire une colle biologique stable et coagulant en moins de 5 minutes par simple addition d'ions calcium.

On a constaté que la préactivation de la phase contact de la coagulation n'active que très peu les plaquettes : moins de 10 % des plaquettes sont activées. On peut donc utiliser comme produit de départ soit un plasma sans plaquettes ou pauvre en plaquettes, soit un plasma contenant des plaquettes ou riche en plaquettes.

On a découvert en outre que le plasminogène n'est pas activé en plasmine de façon significative ni au moment de l'activation de la phase contact, ni au cours des opérations consécutives de préparation de la colle biologique. Ainsi, et contrairement à ce que l'on pouvait redouter, il n'y a pas de risque d'une rupture prématurée du collage due à l'activité fibrinolytique de la plasmine.

Dans la présente demande, on désigne par l'expression "colle biologique" une solution aqueuse obtenue par fractionnement ou concentration du plasma sanguin de façon à concentrer le fibrinogène tout en conservant les facteurs de coagulation nécessaires, qui ont été mentionnés ci-dessus, et en éliminant éventuellement au moins une partie des autres protéines plasmatiques lorsqu'on ne désire pas charger excessivement la colle biologique en protéines telles que l'albumine. Le plasma sanguin contient de 2 à 4 g/L de fibrinogène et de 50 à 60 g/L d'albumine. Une colle biologique obtenue selon l'invention peut contenir par exemple au moins 10 g/L, et en particulier au moins 30 g/L, de fibrinogène. De préférence, elle contient moins de 50 g/L d'albumine, notamment moins de 35 g/L, et en particulier moins de 30 g/L.

La colle biologique obtenue selon l'invention peut contenir divers ingrédients secondaires ajoutés, tels que des agents augmentant la viscosité ou des colorants permettant de contrôler visuellement l'application.

Dans le procédé de l'invention, l'obtention proprement dite de la colle biologique, c'est-à-dire l'obtention d'un concentré de fibrinogène contenant des facteurs de coagulation, est effectuée notamment par toute méthode de fractionnement connue, en particulier par précipitation fractionnée selon les méthodes connues à l'aide d'un agent précipitant tel que par exemple l'éthanol ou le polyéthylèneglycol. Bien entendu, pour chaque agent précipitant susceptible d'être utilisé, il convient de vérifier préalablement que les facteurs de coagulation nécessaires se trouvent dans les mêmes fractions que le fibrinogène. Pour cela, il suffit en pratique de vérifier que le concentré de fibrinogène obtenu est capable de coaguler en moins de 5 minutes par simple addition d'ions calcium, ce qui ne nécessite que des expériences de routine.

Une autre méthode connue consiste à préparer un cryoprécipité. Pour cela, on congèle le plasma, par exemple à température inférieure ou égale à -20°C, puis on décongèle le produit congelé obtenu, par exemple à une température de 0 à 5°C, et en particulier de 2 à 4°C. On obtient ainsi un liquide plasmatique et un produit insoluble appelé cryoprécipité qui est constitué essentiellement de fibrinogène et de divers facteurs de coagulation. Ce cryoprécipité peut être séparé par centrifugation, ou encore par filtration ; voir par exemple le document FR-2.718.033.

Une autre méthode connue consiste à concentrer le plasma à l'aide d'une membrane laissant passer l'eau et les constituants de faible masse moléculaire tout en retenant les protéines.

L'invention concerne également un dispositif pour la mise en oeuvre du procédé défini ci-dessus, dans le cas où l'activateur est un solide insoluble.

Dans un mode de réalisation, non limitatif, ce dispositif est principalement caractérisé par le fait qu'il comprend un premier conteneur destiné à recevoir le plasma avant et/ou pendant l'activation, un deuxième conteneur destiné à recevoir le plasma après l'activation, des moyens pour transférer de façon stérile le plasma depuis le premier conteneur jusqu'au deuxième conteneur, des moyens pour mettre en contact le plasma avec un activateur de la phase contact et, si désiré, des moyens pour séparer le plasma activé et l'activateur de façon que le plasma recueilli dans le second conteneur soit exempt d'activateur.

Selon un mode de réalisation particulier, lesdits moyens de mise en contact comprennent un tube dans lequel l'activateur est confiné, et lesdits moyens de transfert comprennent d'une part une tubulure reliant un orifice de sortie du premier conteneur à l'une des extrémités du tube, et d'autre part, une tubulure reliant un orifice d'entrée du second conteneur à l'autre extrémité dudit tube, de façon que le plasma soit au contact de l'activateur dans le tube lorsqu'il circule entre le premier conteneur et le second conteneur.

Selon un second mode de réalisation, les moyens pour mettre en contact le plasma avec l'activateur et pour séparer le plasma activé et l'activateur comprennent un compartiment dans lequel est confiné l'activateur, ce compartiment étant situé à l'intérieur du premier conteneur. Le compartiment comporte une paroi perméable aux liquides, ce qui permet à la fois le contact avec l'activateur tant que le plasma séjourne dans le premier conteneur, et la séparation du plasma et de l'activateur lorsqu'on fait passer le plasma préactivé dans le second conteneur.

Dans un autre mode de réalisation, les moyens de mise en contact du plasma avec l'activateur comprennent, à l'intérieur du premier conteneur, une paroi sur laquelle est fixé, par exemple par revêtement, l'activateur, et dans ce cas il n'y a pas lieu de prévoir des moyens pour séparer le plasma activé et l'activateur.

L'invention a également pour objet un procédé d'utilisation de la colle biologique telle que définie ci-dessus. Ce procédé est caractérisé par le fait que l'on ajoute à ladite colle une solution aqueuse contenant des ions calcium, sans ajouter de thrombine. On peut ensuite appliquer la colle de façon connue sur le site opératoire ou sur la blessure que l'on désire traiter.

Les ions calcium sont ajoutés à la colle biologique notamment sous la forme d'une solution aqueuse, par exemple une solution aqueuse de chlorure de calcium. La quantité d'ions calcium ajoutée est une quantité suffisante pour que le produit obtenu contienne des ions calcium "libres", c'est-à-dire non chélatés par l'agent complexant. Cette opération est appelée "recalcification". La quantité d'ions calcium libres doit être une quantité suffisante pour permettre la coagulation de la colle biologique dans des délais suffisamment courts. Cette quantité peut être déterminée préalablement par de simples expériences de routine.

Les exemples suivants illustrent l'invention.

### EXEMPLE 1

Le plasma sanguin citraté utilisé comme produit de départ est un plasma riche en plaquettes, obtenu par centrifugation de sang total à 2200xg pendant 6 minutes. Ce plasma contient du citrate de sodium (17 mM).

On ajoute 2 cm³ d'une solution 0,1 M de sulfate de zinc dans 270 cm³ dudit plasma refroidi à 4°C. On fait circuler le plasma dans une colonne de poly(chlorure de vinyle) contenant 10 cm³ de granules de corail de granulométrie 630-1000 µm (INOTEB, ST-GONNERY, FRANCE). Les dimensions de la colonne sont les suivantes : diamètre : 1,4 cm ; hauteur : 14 cm.

La colonne est disposée verticalement, son orifice supérieur étant relié par une tubulure à une poche contenant le plasma sanguin additionné de sulfate de zinc. Ladite poche est située au-dessus de la colonne et le plasma circule en s'écoulant par gravité. L'extrémité inférieure de la colonne comporte un filtre permettant de retenir les particules de corail. Cette extrémité inférieure est reliée par une tubulure à une poche souple permettant de recueillir le plasma préactivé.

La durée de passage à travers la colonne de la totalité du plasma est de 45 minutes.

On dose la kallicréine dans le plasma de la façon suivante : dans un puits d'une plaque de microtitration, on introduit 50 microlitres de tampon véronal-acétate de sodium de pH 7,35 (Diagnostica Stago, Réf.360), et on ajoute soit 50 microlitres d'une solution étalon de kallicréine (CHROMOGENIX, SUEDE, Réf.820845) soit 50 microlitres de l'échantillon à doser. On porte à 37°C et ajoute alors 100 microlitres d'une solution 1,04 mM de substrat S-2302 (CHROMOGENIX, Réf.820340) préchauffé à 37°C. On mesure la variation de densité optique sur une minute, à 405 nm et on en déduit l'activité de kallicréine en comparant cette variation de la densité optique avec celle observée pour la kallicréine étalon.

La teneur en kallicréine du plasma après passage sur les granules de corail est de 20 unités par litre.

Le plasma est ensuite congelé à -30°C, puis décongelé pendant 24 heures dans une enceinte à +4°C. On centrifuge (2000×g) le plasma obtenu. Le culot de centrifugation, c'est-à-dire le cryoprécipité, est recueilli et chauffé à 37°C.

Le temps de coagulation du produit obtenu (cryoprécipité) est déterminé par thromboélastographie, à l'aide du paramètre "r+k" ; voir par exemple MALLETT S.V. et COX D.J.A., British Journal of Anaesthesia, 69:307-313 (1992). Pour cela, on introduit dans la cuve du thromboélastographe (HELLIGE) 175 µL de cryoprécipité à 37°C. On ajoute, à 37°C, 175 µL d'une solution aqueuse 36 mM de chlorure de calcium. L'appareil enregistre automatiquement le tracé de thromboélastographie. On mesure sur le tracé la valeur du paramètre "r+k". Etant donné que le papier sur lequel est enregistré le tracé se déplace à une vitesse de 2 mm par minute, le temps de coagulation en minutes est égal à la moitié de la longueur de "r+k" exprimée en mm.

Le temps de coagulation trouvé est de 2 minutes.

Le cryoprécipité peut aussi être conservé par congélation, puis décongelé au moment de l'utilisation.

Un cryoprécipité préparé comme indiqué ci-dessus, soit récemment obtenu, soit conservé par congélation puis décongelé, est stable pendant plusieurs heures à une température de 20°C : on n'observe aucune coagulation spontanée.

Le cryoprécipité obtenu peut aussi être conservé par lyophilisation.

### EXEMPLE 2

On opère comme à l'exemple 1 à l'exception du fait que les granules de corail sont remplacés par 14 g de billes de verre de diamètre 200-300 µm (SIGMA, Réf. G.1277, L'ISLE D'ABEAU, FRANCE). La durée de passage du plasma à travers la colonne de billes de verre est de 30 minutes. La teneur en kallicréine du plasma après passage sur les billes de verre est de 19 unités par litre.

Le temps de coagulation du cryoprécipité, déterminé par thromboélastographie, est de 3 minutes.

### EXEMPLE 3

On opère de façon similaire à celle décrite à l'exemple 1, en remplaçant les granules de corail par de la poudre de kaolin (SIGMA, Réf. K. 7375). Divers essais ont été effectués en utilisant comme produit de départ soit un plasma riche en plaquettes (PRP) soit un plasma pauvre en plaquettes (PPP).

Les PRP et les PPP ont été obtenus comme indiqué ci-après.

Le PRP est obtenu par centrifugation à 2200×g pendant 6 minutes du sang prélevé.

Le PPP est obtenu par centrifugation à 2500×g pendant 15 minutes.

Pour les cryoprécipités obtenus au départ de PRP, le temps de coagulation est de 100±30 secondes (moyenne de quatre essais).

Pour les cryoprécipités obtenus au départ de PPP, le temps de coagulation est de 190±35 secondes (moyenne de trois essais).

### EXEMPLE 4

On opère de façon analogue à celle décrite à l'exemple 1, sur divers échantillons de plasma riche en plaquettes.

Les résultats sont résumés dans le tableau suivant.

**TABLEAU I**

| Echantillon n° | | Kallicréine (unités/litre) | Temps de coagulation (min) |
|---|---|---|---|
| | (a) | 2,9 | |
| **1** | (b) | 19,7 | |
| | (c) | | 3,25 |
| | (a) | 3,6 | |
| **2** | (b) | 32,3 | |
| | (c) | | 2,75 |
| | (a) | 4,5 | |
| **3** | (b) | 44,3 | |
| | (c) | | 3 |
| (a) plasma de départ | | | |
| (b) plasma activé (après passage sur l'activateur) | | | |
| (c) cryoprécipité | | | |

Dans les expériences suivantes (échantillons n° 4 à 9), on a déterminé en outre le nombre de plaquettes dans le plasma de départ, et on a dosé la plasmine aux divers stades du procédé. Les résultats sont résumés dans le tableau II.

**TABLEAU II**

| Echantillon n° | | Plaquettes (milliers par mm³) | Kallicréine (unités/litre) | Plasmine * | Temps de coagulation (min) |
|---|---|---|---|---|---|
| | (a) | 37 | 3,1 | 0 | |
| **4** | (b) | | 67,7 | 0,33 | |
| | (c) | | | 0,20 | 2,25 |
| | (a) | 368 | 7,1 | 0 | |
| **5** | (b) | | 67,5 | 0,35 | |
| | (c) | | | 0,31 | 4 |
| | (a) | 23 | 3,3 | 0 | |
| **6** | (b) | | 34,8 | 0,20 | |
| | (c) | | | 0 | 2,5 |
| | (a) | 33 | 5,5 | 0 | |
| **7** | (b) | | 29,8 | 0,41 | |
| | (c) | | | 0,22 | 2,5 |
| | (a) | 421 | 7,9 | 0 | |
| **8** | (b) | | 97,6 | 0,42 | |
| | (c) | | | 0,10 | 2,5 |
| | (a) | 261 | 5,6 | 0 | |
| **9** | (b) | | 29,8 | 0,20 | |
| | (c) | | | 0,51 | 2,5 |

| | | | | | |
|---|---|---|---|---|---|
| * % de plasminogène transformé en plasmine | | | | | |

La signification de (a), (b), (c) est donnée après le Tableau I ci-dessus.

On constate que le plasminogène n'est pas activé de façon significative en plasmine, ni au moment de l'activation du système contact, ni au cours de la préparation du cryoprécipité.

### Etude de la libération de fibrinopeptide A

On sait que le fibrinogène est un dimère dont la molécule monomère comporte trois chaînes A α , B β et γ (la masse moléculaire totale de ce monomère est de l'ordre de 170.000). Sous l'action de la thrombine, cette molécule est partiellement hydrolysée, avec libération du fibrinopeptide A (fp A) et du fibrinopeptide B (fpB), à raison d'une molécule de fpA et d'une molécule de fpB par molécule de monomère. Les molécules ainsi débarrassées des fpA et fpB constituent les monomères de fibrine qui s'associent entre eux par des liaisons hydrogène et forment alors la fibrine soluble. La colle biologique de l'invention est pratiquement exempte de fibrine, comme le montre le dosage des fpA par exemple à l'aide du kit de dosage Asserachrom® (STAGO).
Teneur en fibrinogène de la colle biologique : 50g/l (moyenne de 5 préparations).
Teneur en fpA : 365 ng/ml (moyenne de 5 préparations).

La teneur en fpA dans le plasma est de l'ordre de 0 à 2,3 ng/ml, et celle d'un concentré plaquettaire standard est de l'ordre de 14 ng/ml (Bode et Miller, Vox Sanguinis 51, 192-196, 1986).

La masse moléculaire du fpA étant de l'ordre de 1500, on peut calculer aisément que la proportion pondérale de fibrinogène ayant été hydrolysée avec libération de fpA est de l'ordre de 0,08%. Cela prouve que la colle biologique de l'invention est pratiquement exempte de fibrine, et donc aussi de thrombine.

### EXEMPLE 5

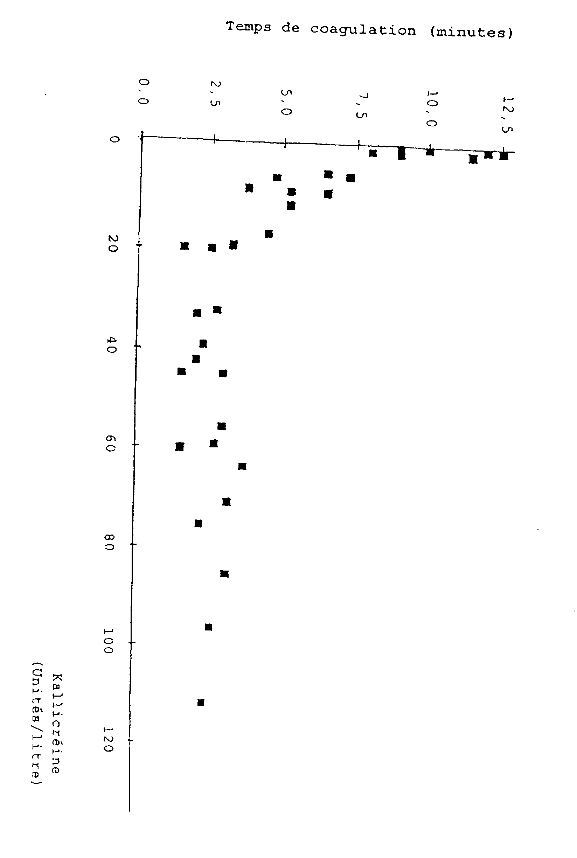

On opère comme à l'exemple 1, en faisant varier le temps de contact avec l'activateur, et on dose à chaque fois la teneur en kallicréine du plasma après activation. On constate que la teneur en kallicréine augmente avec la durée du contact avec l'activateur. On prépare ensuite un cryoprécipité avec chaque plasma étudié, puis on mesure les temps de coagulation obtenus après recalcification. Les résultats sont résumés sur la figure 1 annexée, qui représente le temps de coagulation du cryoprécipité en fonction de la teneur en kallicréine du plasma correspondant après activation. On voit que le temps de coagulation est inférieur à 5 minutes lorsque la teneur en kallicréine est au moins égale à 20 UI/L environ. Une activation plus importante (grâce à une plus longue durée de contact avec l'activateur) conduit à une augmentation de la teneur du plasma en kallicréine, mais n'influence pas le temps de coagulation.

## Revendications

1. Procédé de préparation d'une colle biologique qui, en solution aqueuse, ne coagule pas de façon spontanée pendant au moins une heure à une température de 20°C, et qui est capable de coaguler en moins de 5 minutes par simple addition d'ions calcium, comprenant les étapes consistant à : a) obtenir un plasma sanguin contenant un agent capable de complexer les ions calcium, b) obtenir à partir dudit plasma, selon les méthodes connues, une colle biologique à base de fibrinogène et de facteurs de coagulation, et c) si désiré, soumettre la colle biologique obtenue à une lyophilisation,
dans lequel avant ou pendant l'étape b), on met en contact ledit plasma avec un activateur de la phase contact.

2. Procédé selon la revendication 1, **caractérisé par le fait que** l'agent capable de complexer les ions calcium est le citrate de sodium.

3. Procédé selon la revendication 1 ou 2, **caractérisé par le fait que** la durée de contact avec l'activateur est une durée suffisante pour que le plasma ait une teneur en kallicréine d'au moins 15 unités par litre, ladite unité représentant la quantité de kallicréine capable d'hydrolyser une solution 0,52 mM de D-Prolyl-L-Phénylalanyl-L-Arginine-p-nitro-anilide en solution tampon véronal-acétate de sodium de pH 7,35, à 37°C, à la vitesse initiale de 1 µmol/min.

4. Procédé selon l'une des revendications 1 à 3, **caractérisé par le fait que** ledit activateur est un activateur solide insoluble.

5. Procédé selon la revendication 4, **caractérisé par le fait que** ledit activateur solide est choisi parmi le kaolin, le verre, la célite, le carbonate de calcium, le corail, l'oxyde de zinc et le carbonate de zinc.

6. Procédé selon l'une quelconque des revendications 4 et 5, **caractérisé par le fait que** ledit activateur contient un activateur soluble insolubilisé sur un support solide.

7. Procédé selon l'une quelconque des revendications 4 à 6, **caractérisé par le fait qu'**après un temps de contact suffisant, on sépare et élimine l'activateur.

8. Procédé selon l'une quelconque des revendications 4 à 7, **caractérisé par le fait que** l'on met en contact le plasma avec l'activateur dans un conteneur muni d'un filtre permettant de laisser passer le plasma tout en retenant l'activateur, de façon à séparer le plasma activé de l'activateur après l'étape de mise en contact.

9. Procédé selon l'une quelconque des revendications 4 à 7, **caractérisé par le fait que** l'on effectue ladite mise en contact en faisant circuler ledit plasma dans une colonne contenant l'activateur.

10. Procédé selon la revendication 9, **caractérisé par le fait que** l'on fait circuler ledit plasma dans la colonne jusqu'à un orifice de sortie, ledit orifice de sortie étant muni d'une filtre permettant de retenir l'activateur.

11. Procédé selon l'une quelconque des revendications 4 à 6, **caractérisé par le fait que** l'on met en contact le plasma et l'activateur dans un conteneur ayant une paroi interne sur laquelle est fixé l'activateur.

12. Procédé selon l'une des revendications 1 à 3, **caractérisé par le fait que** ledit activateur est un activateur soluble.

13. Procédé selon la revendication 6 ou 12, **caractérisé par le fait que** ledit activateur soluble est choisi parmi le sulfate de dextran et l'acide ellagique.

14. Procédé selon l'une quelconque des revendications 1 à 13, **caractérisé par le fait que** l'étape b) comprend la préparation d'un cryoprédpité selon les méthodes connues, le cryoprécipité ainsi obtenu constituant ladite colle.

15. Procédé selon l'une quelconque des revendications 1 à 13, **caractérisé par le fait que** l'étape b) est effectuée par précipitation fractionnée.

16. Procédé selon la revendication 15, **caractérisé par le fait que** l'on effectue ladite précipitation fractionnée à l'aide d'un agent précipitant choisi parmi l'éthanol et le polyéthylèneglycol.

17. Dispositif pour la mise en oeuvre du procédé de l'une des revendications 1 à 16, **caractérisé par le fait qu'**il comprend un premier conteneur destiné à recevoir le plasma avant et/ou pendant l'activation, un deuxième conteneur destiné à recevoir le plasma après activation, des moyens pour transférer de façon stérile le plasma depuis le premier conteneur jusqu'au deuxième conteneur, des moyens pour mettre en contact le plasma avec un activateur de la phase contact et, si désiré, des moyens pour séparer le plasma activé et l'activateur de façon que le plasma recueilli dans le second conteneur soit exempt d'activateur.

18. Dispositif selon la revendication 17, **caractérisé par le fait que** lesdits moyens de mise en contact comprennent un tube dans lequel est confiné l'activateur, et lesdits moyens de transfert comprennent d'une part une tubulure reliant un orifice de sortie du premier conteneur à l'une des extrémités du tube, et d'autre part, une tubulure reliant un orifice d'entrée du second conteneur à l'autre extrémité dudit tube, de façon que le plasma soit au contact de l'activateur dans le tube lorsqu'il circule entre le premier conteneur et le second conteneur.

19. Dispositif selon la revendication 17, **caractérisé par le fait que** lesdits moyens de mise en contact avec l'activateur comprennent, à l'intérieur du premier conteneur, une paroi sur laquelle est fixé l'activateur.

20. Dispositif selon la revendication 17, **caractérisé par le fait que** lesdits moyens pour mettre en contact le plasma avec l'activateur et pour séparer le plasma activé et l'activateur comprennent un compartiment dans lequel est confiné l'activateur, ledit compartiment étant situé à l'intérieur du premier conteneur et comportant une parol perméable aux liquides.

21. Procédé d'utilisation de la colle biologique obtenue selon le procédé de l'une quelconque des revendications 1 à 16, **caractérisé par le fait que** l'on ajoute à ladite colle une solution aqueuse contenant des ions calcium, sans ajouter de thrombine.

## Patentansprüche

1. Verfahren zur Herstellung eines biologischen Klebstoffs, der in wässriger Lösung während einer Zeitspanne von mindestens einer Stunde bei einer Temperatur von 20 °C nicht spontan gerinnt, und der durch einfache Zugabe von Calcium-Ionen in weniger als 5 Minuten zu gerinnen vermag, umfassend die folgenden Schritte: a) Gewinnung eines Blutplasmas, das ein Agens zur Komplexierung von Calcium-Ionen enthält, b) Gewinnung eines biologischen Klebstoffs auf der Basis von Fibrinogen und Gerinnungsfaktoren nach bekannten Methoden ausgehend von dem Plasma, und c) gegebenenfalls Gefriertrocknen des gewonnenen biologischen Klebstoffs,
wobei das Plasma vor oder während Schritt b) mit einem Aktivator der Kontaktphase in Kontakt gebracht wird.

2. Verfahren gemäß Anspruch 1, **dadurch gekennzeichnet, dass** das Agens zur Komplexierung von Calcium-Ionen Natriumcitrat ist.

3. Verfahren gemäß Anspruch 1 oder 2, **dadurch gekennzeichnet, dass** die Dauer des Kontakts mit dem Aktivator ausreichend ist, damit das Plasma einen Kallikrein-Gehalt von mindestens 15 Einheiten pro Liter erreicht, wobei die Einheit die Menge an Kallikrein darstellt, die eine 0,52 mM Lösung von D-Prolyl-L-Phenylalanyl-L-Arginin-p-nitroanilid in Veronal/Natriumacetat-Pufferlösung eines pH-Werts von 7.35 bei 37 °C mit einer Anfangsgeschwindigkeit von 1 µmol/min zu hydrolysieren vermag.

4. Verfahren gemäß einem der Ansprüche 1 bis 3, **dadurch gekennzeichnet, dass** der Aktivator ein unlöslicher Feststoffaktivator ist.

5. Verfahren gemäß Anspruch 4, **dadurch gekennzeichnet, dass** der Feststoffaktivator ausgewählt ist, aus Kaolin, Glas, Celite, Calciumcarbonat, Koralle Zinkoxid und Zinkcarbonat.

6. Verfahren gemäß einem der Ansprüche 4 oder 5, **dadurch gekennzeichnet, dass** der Aktivator einen löslichen, auf einem festen Träger unlöslich gemachten Aktivator enthält.

7. Verfahren gemäß einem der Ansprüche 4 bis 6, **dadurch gekennzeichnet, dass** der Aktivator nach einer ausreichenden Kontaktzeit abgetrennt und entfernt wird.

8. Verfahren gemäß einem der Ansprüche 4 bis 7, **dadurch gekennzeichnet, dass** das Plasma mit dem Aktivator in einem Behälter in Kontakt gebracht wird, der mit einem Filter ausgestattet ist, der es erlaubt, das Plasma unter Zurückhalten des Aktivators durchlässt, um das aktivierte Plasma nach dem Schritt des in Kontakt Bringens von dem Aktivator zu trennen.

9. Verfahren gemäß einem der Ansprüche 4 bis 7, **dadurch gekennzeichnet, dass** der Kontakt dadurch hergestellt wird, dass man das Plasma durch eine Säule laufen lässt, die den Aktivator enthält.

10. Verfahren gemäß Anspruch 9, **dadurch gekennzeichnet, dass** man das Plasma durch eine Säule bis zu einer Ausgangsöffnung laufen lässt, wobei die Ausgangsöffnung mite inem Filter ausgestattet ist, der das Zurückhalten des Aktivators erlaubt.

11. Verfahren gemäß einem der Ansprüche 4 bis 6, **dadurch gekennzeichnet, dass** das Plasma mit dem Aktivator in einem Behälter in Kontakt gebracht wird, der eine Innenwand aufweist, auf der der Aktivator fixiert ist.

12. Verfahren gemäß einem der Ansprüche 1 bis 3, **dadurch gekennzeichnet, dass** der Aktivator ein löslicher Aktivator ist.

13. Verfahren gemäß Anspruch 6 oder 12, **dadurch gekennzeichnet, dass** der lösliche Aktivator ausgewählt ist aus Dextransulfat und Ellagsäure.

14. Verfahren gemäß einem der Ansprüche 1 bis 13, **dadurch gekennzeichnet, dass** der Schritt b) die Herstellung eines Kryopräzipitats nach bekannten Methoden umfasst, wobei das auf diese Weise erhaltene Kryopräzipitat den Klebstoff darstellt.

15. Verfahren gemäß einem der Ansprüche 1 bis 13, **dadurch gekennzeichnet, dass** der Schritt b) durch fraktionierte Fällung erfolgt.

16. Verfahren gemäß Anspruch 15, **dadurch gekennzeichnet, dass** die fraktionierte Fällung mittels eines Fällungsmittels, ausgewählt aus Ethanol und Polyethylenglycol, erfolgt.

17. Vorrichtung zur Durchführung des Verfahrens nach einem der Ansprüche 1 bis 16, **dadurch gekennzeichnet, dass** sie einen ersten Behälter zur Aufnahme des Plasmas vor und/oder während der Aktivierung umfasst, einen zweiten Behälter zur Aufnahme des Plasmas nach der Aktivierung, Einrichtungen, um das Plasma unter sterilen Bedingungen vom ersten in den zweiten Behälter zu Überführen, Einrichtungen, um das Plasma mit einem Aktivator der Kontaktphase in Kontakt zu bringen und gegebenenfalls Einrichtungen, um das aktivierte Plasma und den Aktivator voneinander so zu trennen, dass das in dem zweiten Behälter aufgefangene Plasma frei von Aktivator ist.

18. Vorrichtung gemäß Anspruch 17, **dadurch gekennzeichnet, dass** die Einrichtungen zum Kontaktieren ein Rohr umfassen, in das der Aktivator eingebracht ist, und die Einrichtungen zum Überführen einerseits einen Schlauch umfassen, der eine Ausgangsöffnung des ersten Behälters mit einem Ende des Rohrs verbindet, und andererseits einen Schlauch, der eine Eingangsöffnung des zweiten Behälters mit dem anderen Ende des Rohrs verbindet, so dass das Plasma mit dem Aktivator in dem Rohr in Kontakt tritt, wenn es vom ersten Behälter in den zweiten fließt.

19. Vorrichtung gemäß Anspruch 17, **dadurch gekennzeichnet, dass** die Einrichtungen zum Kontaktieren mit dem Aktivator eine Wand im Inneren des ersten Behälters umfassen, auf der der Aktivator fixiert ist.

20. Vorrichtung gemäß Anspruch 17, **dadurch gekennzeichnet, dass** die Einrichtungen, um das Plasma mit dem Aktivator in Kontakt zu bringen und um das aktivierte Plasma und den Aktivator voneinander zu trennen ein Kompartiment umfassen, in das der Aktivator eingebracht ist, wobei das Kompartiment im Inneren des ersten Behälters liegt und eine Wand umfasst, die durchlässig für Flüssigkeiten ist.

21. Verfahren zur Anwendung des biologischen Klebstoffs, erhalten nach dem Verfahren gemäß einem der Ansprüche 1 bis 16, **dadurch gekennzeichnet, dass** zu dem Klebstoff eine wässrige, Calcium-Ionen enthaltende Lösung gegeben wird, ohne Thrombin zuzufügen.

## Claims

1. A method for preparing a biological glue which, in aqueous solution, does not coagulate spontaneously for at least one hour at a temperature of 20°C, and which is able to coagulate in less than 5 minutes by merely adding calcium ions, comprising the steps of: a) obtaining blood plasma containing an agent capable of complexing calcium ions, b) obtaining from said plasma, by known methods, a biological glue based on fibrinogen and coagulation factors, and c) if desired, subjecting the resulting biological glue to freeze-drying,
wherein, before and/or during step b), said plasma is contacted with a contact phase activator.

2. The method according to claim 1, **characterized in that** the agent capable of complexing calcium ions is sodium citrate.

3. The method according to claim 1 or 2, **characterized in that** the plasma is contacted with the activator for a time sufficiently long to provide a plasma having a kallikrein content of at least 15 units per liter, said unit representing the amount of kallikrein capable of hydrolyzing a 0.52 mM solution of D-prolyl-L-phenylalanyl-L-arginine-p-nitro-anilide in a veronal-sodium acetate buffer solution with a pH of 7.35, at 37°C, at the initial rate of 1 µmol/min.

4. The method according to one of claims 1 to 3, **characterized in that** said activator is an insoluble solid activator.

5. The method according to claim 4, **characterized in that** said solid activator is selected from kaolin, glass, Celite, calcium carbonate, coral, zinc oxide and zinc carbonate.

6. The method according to any one of claims 4 and 5, **characterized in that** said activator contains a soluble activator insolubilized on a solid substrate.

7. The method according to any one of claims 4 to 6, **characterized in that** the activator is separated and eliminated after a sufficiently long contact time.

8. The method according to any one of claims 4 to 7, **characterized in that** the plasma is contacted with the activator in a container having a filter that allows the plasma to pass through while holding back the activator in order to separate the activated plasma from the activator after the contacting step.

9. The method according to any one of claims 4 to 7, **characterized in that** said contact is effected by circulating said plasma in a column containing the activator.

10. The method according to claim 9, **characterized in that** said plasma is circulated in the column up to an outlet opening, said outlet opening having a filter for holding back the activator.

11. The method according to any one of claims 4 to 6, **characterized in that** the plasma and the activator are brought into contact in a container having an inner wall to which the activator is fixed.

12. The method according to one of claims 1 to 3, **characterized in that** said activator is a soluble activator.

13. The method according to claim 6 or 12, **characterized in that** said soluble activator is selected from dextran sulfate and ellagic acid.

14. The method according to any one of claims 1 to 13, **characterized in that** step b) comprises the preparation of a cryoprecipitate by known methods, which cryoprecipitate thus obtained consists of said glue.

15. The method according to any one of claims 1 to 13, **characterized in that** step b) is carried out by fractional precipitation.

16. The method according to claim 15, **characterized in that** said fractional precipitation is carried out with the aid of a precipitating agent selected from ethanol and polyethylene glycol.

17. A device for implementing the method of one of claims 1 to 16, **characterized in that** it comprises a first container designed to receive the plasma before and/or during activation, a second container designed to receive the plasma after activation, means for transferring, in a sterile fashion, the plasma from the first container to the second container, means for bringing the plasma into contact with the contact phase activator, and, if desired, means for separating the activated plasma from the activator so that the plasma collected in the second container is activator-free.

18. The device according to claim 17, **characterized in that** said contacting means comprises a tube in which the activator is confined, and said transfer means comprise, on the one hand, a pipe connecting an outlet opening of the first container with one end of the tube, and, on the other hand, a pipe connecting an inlet opening of the second container with the other end of said tube, such that the plasma is in contact with the activator in the tube when the plasma circulates between the first container and the second container.

19. The device according to claim 17, **characterized in that** said means for bringing the plasma into contact with the activator comprise a wall within the first container to which the activator is fixed.

20. The device according to claim 17, **characterized in that** said means for bringing the plasma into contact with the activator and said means for separating the activated plasma from the activator comprise a compartment in which the activator is confined, said compartment being located within the first container and having a wall permeable to liquids.

21. A method for using the biological glue obtained according to the method of any one of claims 1 to 16, **characterized in that** an aqueous solution containing calcium ions is added to said glue, without adding thrombin.
